(19) Europäisches Patentamt
European Patent Office
Office européen des brevets

(11) **EP 2 239 116 A2**

(12) **EUROPÄISCHE PATENTANMELDUNG**

(43) Veröffentlichungstag:
**13.10.2010 Patentblatt 2010/41**

(51) Int Cl.:
***B27G 1/00*** *(2006.01)* ***G01N 21/898*** *(2006.01)*

(21) Anmeldenummer: **10003799.3**

(22) Anmeldetag: **09.04.2010**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA ME RS**

(30) Priorität: **09.04.2009 DE 102009016733**

(71) Anmelder: **Baumer Innotec AG**
**8500 Frauenfeld (CH)**

(72) Erfinder: **Eberhardt, Joerg**
**88074 Sibratshausen (DE)**

(74) Vertreter: **Schmidt, Oliver**
**Blumbach & Zinngrebe**
**Alexandrastrasse 5**
**65187 Wiesbaden (DE)**

(54) **Verfahren und Vorrichtung zum automatischen Ausbessern von Paneelen**

(57) Die Erfindung betrifft ein Verfahren und eine Vorrichtung zum automatischen Flicken von Fehlstellen, welch eine geometrisch genauen und hochempfindlichen Lokalisierung der Fehlstellen auch bei nicht-ebenen Paneelen ermöglicht.

Fig. 1

EP 2 239 116 A2

# EP 2 239 116 A2

## Beschreibung

Bei der Herstellung von Paneelen, insbesondere aus Holzwerkstoffen mit einer sichtbaren Oberfläche aus Naturholz wie z.B. Tischlerplatten, Sperrholzplatten, Fertigparkett, Möbelplatten usw. ist die Oberfläche sehr häufig durch die für das Naturprodukt "Holz" typische Fehlstellen wie kranke Äste, Harzgallen, Risse, Faulstellen aber auch konstruktionsbedingte Fehler, wie offene Verbindunsgspalten usw. gestört. Es ist daher üblich, diese Stellen in den oft sehr grossen Paneelen (mit Abmessungen bis zu 6m x 2m ) entweder manuell oder automatisch zu flicken, um die Verluste durch schlechte Oberflächen zu begrenzen und den Werkstoff möglichst gut zu nutzen.

Hierbei wird die Fehlstelle visuell lokalisiert, ausgefräst oder ausgestanzt und durch ein Flickmittel wie Spachtelmasse oder auch Dübel aufgefüllt. Die manuelle Durchführung dieser Aufgabe erfordert eine große Anzahl von Mitarbeitern und stellt sowohl einen hohen Kosten- und Zeitfaktor dar, welchen man reduzieren möchte.

In der US 4,614,555 wird ein System für das automatische Flicken von Sperrholz-Paneelen vorgestellt, wobei die elektrooptische Abtastung zur Fehlerlokalisierung nur summarisch beschrieben und die Problematik der geometrischen Genauigkeit der Defektposition nicht angesprochen wird.

In der US 4,984,172 wird eine automatische Flickstation, ebenfalls für Sperrholzplatten beschrieben, bei welcher die optische Fehler-Erkennung und -Lokalisierung mit Hilfe einer Anordnung von Linienbeleuchtung (typischerweise einer Laserlinie) und einer CCD-Matrixkamera in einer Triangulations-Anordnung durchgeführt wird. Die Linienbeleuchtung beleuchtet aus einer senkrechten Position zur Paneel-Oberfläche diese mit einer hellen Linie; diese Linie wird mit einer Matrixkamera unter einem steilen Winkel bezogen auf die Paneel-Oberfläche erfasst. Diese Anordnung stellt eine bekannte 3D-Laserlinientriangulation dar und erlaubt es, lokale 3D-Fehler in ihrer XY-Position und in ihrer Tiefe/Höhe Z auszumessen. Um in Astlöcher hinein messen zu können, muß der Beobachtungswinkel der Kamera bezogen auf die Oberflächen des Paneels daher steil sein, typischerweise 60 bis 70 Grad. Es wird ausserdem beschrieben, dass aus der Intensität des reflektierten Lichtes der Laserlinie auf das Vorhandensein von ebenen Defekten geschlossen werden kann.

Dieses Verfahren ist durch eine Reihe von Nachteilen gekennzeichnet:

a) die kombinierte Erfassung von 2D- und 3D Fehler mit einer Triangulationsanordnung erfordert prinzipbedingt die Verwendung einer Matrixkamera, welches eine Einschränkung der Meßgeschwindigkeit im Vergleich zur Verwendung von den prinzipiell schneller taktbaren Zeilenkameras bedeutet.

b) wird das Paneel über die gesamte Breite optisch erfasst, werden bei großen Paneelen zahlreiche nebeneinander angeordnete Matrixkameras benötigt, deren Bildfelder sich aneinander anschliessen oder sich leicht überlappen. Dies ist ebenfalls ein nachteiliger Kostenfaktor im Vergleich zum Einsatz einer einzelnen oder nur einiger weniger hochauflösender Zeilenkameras und ein schwieriger mechanisch zu realisierender Aufbau.

c) die Fehler der Holzoberfläche werden infolge der Lasertriangulations-Anordnung unter einem steilen Winkel (bezogen auf die Paneel-Ebene) beobachtet. Diese Anordnung von Beleuchtung und Kamera stellt für die Fehler, welche sich durch eine unterschiedliche lokale Reflektion des Lichtes darstellen, eine ungünstig unempfindliche Anordnung dar. Viele Oberflächenfehler wie Glanz- und Mattstellen, aufgeraute Stellen, Rattermarken, dünne Risse usw. können nicht oder nur schlecht erkannt werden, da die Holzstruktur kaum durch solche Fehler beeinflusst wird.

d) Zudem wird auch die Problematik der Fehlererkennung bei unebenen Paneelen nicht gelöst. Häufig sind Paneele in Quer- und Längsrichtung gewellt, wobei diese Welligkeit tolerierbar ist und keinen zu reparierenden Fehler darstellt. Allerdings sollten solche Welligkeiten keinen Einfluß auf die Genauigkeit der Lokalisierung der Defekten haben.

Es besteht daher ein hohes technisches und wirtschaftliches Interesse an einem auch bei nicht ebenen Paneelen geometrisch genauen, hochempfindlichen und preiswerten System für das automatische Flicken von Fehlstellen, insbesondere einem System bei welchem die Erkennung, Lokalisierung und Charakterisierung der zu flickenden Fehler mit Hilfe von preiswerten hochauflösenden Zeilenkameras durchgeführt wird, wobei mit einer flachen Anordnung von gerichteter Beleuchtung und beobachtenden Kameras auch sehr schwach ausgeprägte Oberflächenfehler erkannt werden können und gleichzeitig die geometrisch hochgenaue Bestimmung der Lagekoordinaten der Defekte und der Defektkonturen trotz der vorhandenen Welligkeiten und Wölbungen des Paneels sichergestellt ist.

Dazu ist erfindungsgemäß ein Verfahren zum automatischen Flicken von Defekten in Paneelen vorgesehen, bei welchem die Defekte mit Hilfe eines optischen Bildaufnehmers erkannt und diese Stellen mit Hilfe von zumindest einem rechnergesteuerten Werkzeug ausgefüllt oder beseitigt werden, wobei das rechnergesteuerte Werkzeug anhand der von dem optischen Bildaufnehmer ermittelten Defekt-Positionen angesteuert wird, wobei der optische Bildaufnehmer die Oberfläche des Paneels unter schrägem Winkel betrachtet, und wobei aus dem oder den erzeugten Bildern mittels einer rechnergestützten automatisierten Bildverarbeitung die zu flickenden Defekte erkannt und lokalisiert werden. Zusätzlich wird eine Höhenmessung der Lage der Oberfläche des Paneels mittels einer Höhenmesseinrichtung durchgeführt und die lokale Höhe der Defekte anhand der Höhenmessung bestimmt wird. Die anhand der automatisierten Bildverarbeitung ermittelten lateralen Positionen der Defekte werden mittels der bestimmten lokale Höhe der Defekte korrigiert und das rechnergesteuerte Flickwerkzeug mit diesen korrigierten Positionsinformationen der Defekte angesteuert.

Vorzugsweise werden die zu flickenden Defekte erkannt und lokalisiert, während das Paneel lateral entlang einer Vorschubrichtung relativ zum optischen Bildaufnehmer vorbeigeführt wird. Dies ist günstig, um eine Erfassung großer Paneele zu ermöglichen. Vorzugsweise wird dabei das Paneel bewegt, eine Relativbewegung ist selbstverständlich aber auch durch eine Bewegung des optischen Bildaufnehmers in lateraler Richtung entlang der Oberfläche des Paneels möglich.

Eine entsprechende Vorrichtung zur Durchführung des erfindungsgemäßen Verfahren umfasst dazu

- zumindest einen optischen Bildaufnehmer, der so angeordnet ist, dass dessen Blickrichtung schräg unter einem Winkel zur Oberfläche eines zu untersuchenden Paneels liegt,
- zumindest einen Höhensensor zur Ermittlung der Höhenlage der Oberfläche des Paneels,
- eine Recheneinrichtung zur automatischen Erkennung von Defekten anhand der vom zumindest einen optischen Bildaufnehmer gelieferten Bilddaten und zur Ermittlung der lateralen Position der Defekte, wobei die Recheneinrichtung dazu eingerichtet ist, anhand der vom Höhensensor gelieferten Meßwerte die Höhenlage der Defekte zu bestimmen und die laterale Position der Defekte anhand der bestimmten Höhenlage zu korrigieren, und
- ein Flickwerkzeug, welches von der Recheneinrichtung angesteuert ist, um die Defekte an deren durch die Höhenlage korrigierten lateralen Positionen auszubessern.

Vorzugsweise ist zur Erfassung der Paneeloberfläche im Durchlauf eine Vorschubeinrichtung zum Vorbeiführen des Paneels am optischen Bildaufnehmer entlang einer lateralen Vorschubrichtung vorgesehen, wobei der optische Bildaufnehmer dann dementsprechend so angeordnet ist, dass dessen Blickrichtung schräg zur Vorschubrichtung liegt.

Die Betrachtung der Oberfläche unter schrägem Winkel zur Vorschubrichtung, beziehungsweise entsprechend zur Oberfläche des Paneels |erlaubt es allgemein, auch solche Defekte wahrzunehmen, die unter senkrechter Betrachtung nicht oder allenfalls kaum wahrnehmbar sind. Während eine Betrachtung senkrecht von oben empfindlich auf Farbveränderungen ist, wie sie etwa an Astlöchern auftreten, kann eine schräge Betrachtung auch die Struktur der Oberfläche, beispielsweise Aufrauhungen sichtbar machen, die aber keine Farbveränderung mit sich bringt. Ein Beispiel, an welchem der Vorteil der Erfindung besonders deutlich wird, sind etwa ausgerissene Teile aus einer Holzoberfläche. Senkrecht von oben betrachtet können derartige Defekte leicht übersehen werden, besonders wenn diese nicht derart tief sind, dass sich Schatten bilden. Wird die Oberfläche hingegen unter schrägem Winkel betrachtet, ist ein derartiger Defekt deutlich als Vertiefung sichtbar. Mit anderen Worten ist die Erfindung besonders auf die Oberflächentopographie empfindlich. Die Höhenkorrektur erlaubt dabei dann dennoch die exakte Lokalisierung von Defekten auch bei verformten Paneelen.

Diese Detektionsempfindlichkeit kann dann noch besonders durch eine Hellfeld-Beleuchtung verbessert werden. Hierbei wird in Weiterbildung der Erfindung die zu flickende Oberfläche von mindestens einer Hellfeld-Anordnung von Bildaufnehmer und Beleuchtungsquelle optisch unter schrägem Winkel bezogen auf Oberfläche des Paneels erfasst. Dazu wird eine Beleuchtungseinrichtung vorgesehen, welche so gegenüber dem optischen Bildaufnehmer angeordnet ist, dass die zu flickende Oberfläche vom optischen Bildaufnehmer im Hellfeld des von der Oberfläche reflektierten Strahls betrachtet wird. Oft sind die Paneele je nach Beschaffenheit nur diffus reflektierend. Demgemäß ist es auch nicht erforderlich, dass der Bildaufnehmer und die Beleuchtung den jeweiligen vom Bildaufnehmer betrachteten Oberflächenbereich unter exakt dem gleichen Winkel beleuchten, beziehungsweise erfassen. Vielmehr können für eine Hellfeld-Anordnung auch etwas unterschiedliche Winkel für Beleuchtung und Blickrichtung des Bildaufnehmers eingesetzt werden. Vorzugsweise ist der Unterschied der Winkel der Beleuchtung und der Blickrichtung, gemessen zur Oberfläche oder auch deren Lot aber geringer als 25°, vorzugsweise geringer als 10°.

Vorzugsweise ist der optische Bildaufnehmer unabhängig von der Beleuchtung weiterhin so angeordnet, dass dieser die Oberfläche eines Paneels unter einem Winkel von 5 bis 60, vorzugsweise von 8 bis 30 Grad bezogen auf die Oberfläche betrachtet. Der kleine Winkel zwischen Blickrichtung und Oberfläche des Paneels, beziehungsweise die streifende Betrachtung sind wie gesagt günstig, um eine hohe Empfindlichkeit für Oberflächendefekte zu erhalten, welche die Oberflächentopographie des Paneels verändern.

Es kann ausserdem kostensparend die Tatsache genutzt werden, dass die Welligkeit und Wölbung eines Paneels eine im Vergleich zu den zu flickenden Fehlstellen sehr niederfrequente Störungen darstellen, so dass es oft genügt, an relativ wenigen Meßpositionen die Höhe der Paneeloberfläche mit einem Höhenmesser zu erfassen und Zwischenpositionen der Paneelhöhe durch Interpolation zu ermitteln. Demgemäß wird in Weiterbildung der Erfindung die lokale Höhe der zu flickenden Oberfläche im Durchlauf mit einer Anzahl von optischen Distanzsensoren an diskreten Positionen der zu flickenden Oberfläche erfasst.

Eine hohe Dichte von Höhenmessdaten kann erhalten werden, indem die lokale Höhe der zu inspizierenden Oberfläche mit Hilfe mindestens eines die zu flickende Oberfläche im Durchlauf linienförmig quer zur Vorschubrichtung des Paneels in der Höhe ausmessenden Distanzsensors bestimmt wird.

Unabhängig von der Art und Weise, wie diskrete Höhenmesswerte erhalten werden, kann über eine zweidimensionale Interpolation aus den diskreten Höhenmeßwerten dann eine stetige Höhenkarte der zu flickenden Oberfläche ermittelt werden. Die interpolierten Höhenwerte dieser Höhenkarte können dann zur Lagekorrektur der Defekte verwendet werden.

Das Flickwerkzeug kann vorteilhaft eine Einrichtung zum Aushöhlen, vorzugsweise Ausfräsen oder Ausstanzen oder Ausbohren von Defekten und eine Einrichtung zum Ausfüllen des ausgehöhlten Bereichs durch ein Flickmittel, vorzugsweise durch Spachtelmasse oder Dübel umfassen. Sind beispielsweise aber Oberflächendefekte ausschließlich in Form von Vertiefungen zu beseitigen, kann die Einrichtung zum Aushöhlen auch gegebenenfalls entfallen.

Um ein Bild der Oberfläche des Paneels zu erhalten, wird die zu flickende Oberfläche vorzugsweise durch einen oder mehrere zeilenweise abtastende Bildsensoren im Durchlauf erfasst. Derartige Bildsensoren als Bestandteil des oder der optischen Bildaufnehmer können ein- oder mehrzeilige Zeilen- beziehungsweise Matrixsensoren sein. Auch Multi-Matrix-Sensoren können eingesetzt werden. Im letzteren Fall wird das Bild beispielsweise aus mehreren, von den einzelnen Sensoren gelieferten Teilbildern zusammengesetzt. Selbstverständlich können aber auch allgemein MatrixSensoren als Zeilensensoren eingesetzt werden, indem nur eine Zeile oder ein Zeilenbereich des Sensors zur Erstellung des Bilds verwendet wird. Dies bedeutet, dass einzelne nacheinander aufgenommene Zeilen zum Bild der Oberfläche zusammengesetzt werden. Durch den Vorschub des Paneels werden bei den Aufnahmen der Zeilen sukzessive hintereinanderliegende linienförmige Bereiche des Paneels erfasst und zusammengesetzt. Das Aufnahmeverfahren ähnelt damit dem eines Dokumentenscanners. Gerade bei der Aufnahme unter schrägem Winkel bietet diese Aufnahmetechnik Vorteile gegenüber dem Fotografieren größerer rechteckiger Bereiche der Oberfläche, wie dies typischerweise mit einem Matrix-Sensor geschieht. Wird das Bild aus in fester Entfernung zum Sensor aufgenommenen schmalen, beziehungsweise linienförmigen Bereichen zusammengesetzt, ist die Tiefenschärfe der Optik weniger kritisch. Ansonsten bestände bei der Aufnahme größerer Bereiche des Paneels das Problem, daß die Bereiche innerhalb des Bilds stark unterschiedliche Entfernungen zum Sensor haben. Aufgrund der endlichen Tiefenschärfe könnte so die Bildauflösung nur schwer über den gesamten aufgenommenen Bereich hinweg konstant gehalten werden.

Auch kann die zu flickende Oberfläche durch eine zeilenweise oder linienförmig die Oberfläche beleuchtende Lichtquelle beleuchtet werden. Wird nur eine schmale Linie ausgeleuchtet, kann der Aufnahmebereich in vorteilhafter Weise durch den ausgeleuchteten linienförmigen Bereich bestimmt werden und so eine Aufnahme der gesamten Oberfläche mit konstanter Bildschärfe erfolgen.

Die erfindungsgemäße Erfassung der Paneeloberfläche unter flachem Winkel, beziehungsweise schräg zur Oberfläche kann in Weiterbildung der Erfindung auch mit weiteren Anordnungen zur Aufnahme der Oberfläche kombiniert werden. Insbesondere kann eine Kombination mit einer Aufnahme senkrecht auf die Paneeloberfläche sinnvoll sein, um verschiedenartige Defekte zu erkennen. Ist beispielsweise die Oberfläche des Paneels bereits glänzend, so sind ist im Hellfeld insbesondere Änderungen der Rauhigkeit der dieser Oberfläche sichtbar. Farbliche Veränderungen sind dann aber nur schwer zu erkennen, da das Helligkeitssignal vornehmlich durch das an der Oberfläche reflektierte Licht verursacht wird. Die Farbe wird hingegen sichtbar, wenn nicht reflektiertes, sondern diffus gestreutes Licht betrachtet wird. Demgemäß ist in Weiterbildung der Erfindung vorgesehen, die zu flickende Oberfläche durch mehrere kombinierte Bilderfassungs- und Beleuchtungsanordnungen zur Erkennung von zu flickenden Defekte abzutasten. Die aus den Bilddaten ermittelten Positionsdaten der Defekte und/oder der Defektkonturpunkte können dann insbesondere bei einer solchen Anordnung mit Hilfe von zusätzlich gemessenen Höhenwerten korrigiert werden, bei welcher die Bilderfassungs-/ Beleuchtungsanordnung prinzipbedingt zu Positionsfehlern der Defekte und/oder Defektkonturpunkte bei einer sich ändernden Höhe der Paneeloberfläche führt. Ein Beispiel hierzu ist eine erfindungsgemäße Erfassung des Hellfelds unter schrägem Winkel zur Oberfläche, kombiniert mit einer Erfassung von Streulicht mittels einer weiteren Kamera unter steilem oder rechtem Winkel auf die Oberfläche. Dabei ändern sich prinzipbedingt die ermittelten Ortskoordinaten bei der Erfassung des Hellfelds in Abhängigkeit von der Höhe der Oberfläche und wird entsprechend mit den gemessenen Höhendaten korrigiert. Eine Korrektur der Daten der anderen, senkrecht auf die Oberfläche schauenden Kamera kann entfallen. Es ist möglich, für beide Kameras die gleiche Beleuchtung zu verwenden. Insbesondere bei der Verwendung eines Linienlichts kann so auch sichergestellt werden, dass beide Kameras simultan den gleichen Oberflächenbereich abtasten.

Gemäß noch einer Weiterbildung der Erfindung wird von der rechnergestützten automatisierten Bildverarbeitung eine Charakterisierung der ermittelten Defekte, insbesondere hinsichtlich zumindest einer der Eigenschaften Form, Fläche, Farbe vorgenommen. Es können dann verschiedene für die Eigenschaften spezifischen Grenzwerte gesetzt werden, anhand welchen bestimmt wird, ob ein erkannter Defekt tatsächlich vom Flickwerkzeug auszubessern ist. Ist beispielsweise die Fläche des Defektes kleiner als ein vorgegebener Grenzwert, kann eine Ausbesserung entfallen. Auch können bestimmte Formen von einer Ausbesserung ausgeschlossen werden. So ist es beispielsweise denkbar, dass runde oder ovale Defekte nicht ausgebessert werden sollen, wenn im Erscheinungsbild einer Holzoberfläche des Paneels Astfreiheit nicht verlangt wird.

Generell ist es von Vorteil, wenn die rechnergestützte automatisierte Bildverarbeitung die Kontur eines Defekts bestimmt, um dessen Ausdehnung zu bestimmen und den auszubessernden Bereich festzulegen. In diesem Fall ist es dann auch sinnvoll, nicht nur die Position des Defekts, sondern auch die Lage der einzelnen Konturpunkte des Defekts anhand der mittels der bestimmten lokale Höhe des Defekts oder die aus den Konturpunkten gewonnene Form des Defekts zu korrigieren.

Die Erfindung wird nachfolgend anhand der beigeschlossenen Figuren näher erläutert. Dabei verweisen gleiche Bezugszeichen auf gleiche oder entsprechende Elemente. Es zeigen:

Fig. 1 eine Anordnung zur Lokalisierung von Fehlstellen in Paneelen,

Fig. 2a und 2b eine Meßanordnung zur Detektion von Fehlstellen auf einem Paneel mit senkrecht auf das Paneel blickender Kamera, wobei in Fig. 2a das Paneel in Sollposition und in Fig. 2b in gegenüber der Sollposition erhöhter Lage angeordnet ist.

Fig. 3a und 3b eine im Hellfeld arbeitende Meßanordnung zur Detektion von Fehlstellen auf einem Paneel, wobei in Fig. 3a das Paneel in Sollposition und in Fig. 3b in gegenüber der Sollposition erhöhter Lage angeordnet ist,

Fig. 4 eine symmetrische Hellfeld-Anordnung,

Fig. 5 schematisch eine Seitenansicht einer Anordnung mit punktförmig messendem Distanzsensors, zur Bestimmung der lokalen Höhe entlang der Vorschubrichtung eines gewellten Paneels und eine Hellfeld-Anordnung zur Erfassung von Defekten,

Fig. 6 in Aufsicht ein Paneel mit einer Anordnung von Distanzsensoren, und

Fig. 7 ein errechnetes Höhenprofil eines Paneels.

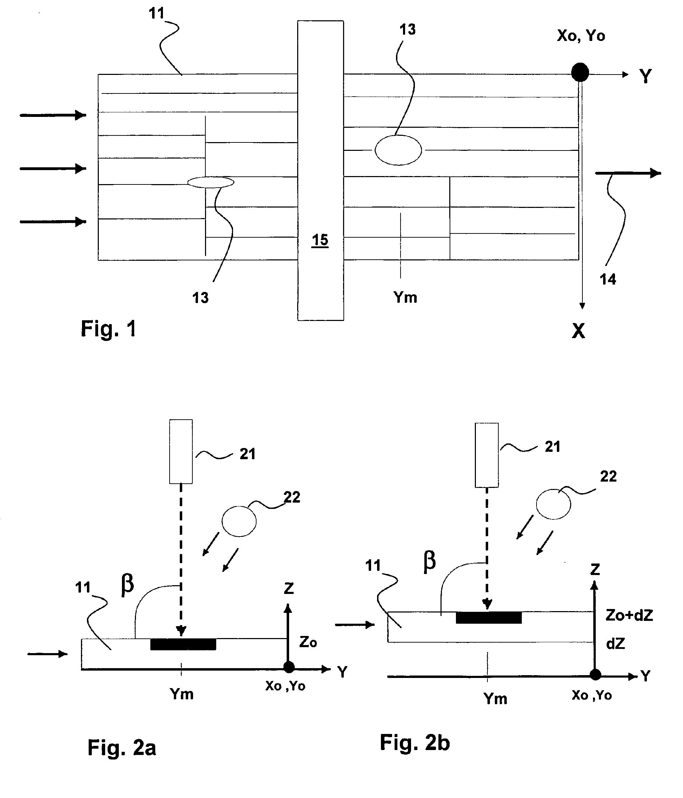

Wie in Fig. 1 skizziert, wird ein Holzpaneel 11 mit Hilfe einer Transportvorrichtung entlang einer Vorschubrichtung 14 durch einen optischen Scanner 15 bewegt, welcher mit Hilfe von Bildverarbeitungstechniken Fehlstellen 13, wie beispielsweise Astlöcher, Faulstellen, Glanzstellen, Risse usw. erkennt und in dem objekt-bezogenen Koordinatensystem $[X_0, Y_0]$ lokalisiert. Hierbei ist Y die Vorschubrichtung und X die Querkoordinate. Die Defekte seien beispielweise anhand ihrer Schwerpunkt-Koordinaten $[Ym, Xm]$ lokalisiert.

Mit den so erzeugten Informationen über den Ort und den Charakter der Fehlstelle kann dann ein in Fig. 1 nicht dargestelltes Werkzeug zum automatischen Flicken numerisch, beziehungsweise rechnergestützt angesteuert werden. Dieses Flicken geschieht vorzugsweise in zwei Bearbeitungsstufen:

a) ein möglichst genaues automatisches Ausfräsen/Ausstanzen/Säubern der Fehlstelle, und
b) ein automatisches Flicken der Fehlstelle durch Einpressen eines Dübels oder einer Spachtelmasse, Auffüllen mit Ein- oder Zwei-Komponentenkleber oder ähnlichen Füllstoffen.

Die zu flickenden Paneele können große Dimensionen annehmen (typ. 6000 mm x 2000 mm); die absolute geometrische Genauigkeit des Flickens sollte aber im Bereich eine Millimeters liegen und sich so genau wie möglich auf die defekte Stellen beschränken. Es wird daher von solchen automatischen Flicksystemen sowohl eine sehr gute Detektion der eigentlichen Fehlstellen als auch eine sehr hohe relative Genauigkeit der Positions- und Formermittlung dieser Fehlstellen von ca. 1:6000 verlangt. Diese hohen geometrischen Genauigkeiten sind um so schwieriger zu erreichen, als oft solche Holzpaneele nicht perfekt eben sind sondern (in der Regel großflächige) Wölbungen und Wellungen aufzeigen, welche nur teilweise durch Vakuumbänder oder mechanische Niederhalter beseitigt werden können.

Die Aufgabe des vorgestellten Erfindungsgedanken ist es, ein kostengünstiges Verfahren und eine Anordnung zu finden, mit welchem die Defekte mit hoher Empfindlichkeit erkannt werden können und gleichzeitig auch bei nicht ebenen Paneelen mit hoher Genauigkeit geometrisch im Paneel-bezogenen Koordinaten-system mit dem Ursprung $[X_0, Y_0, Z_0]$ lokalisiert werden.

Viele Holzpaneele zeigen großflächige Wölbungen und Wellen auf, welche nicht zu Fehler in der Lokalisierung der Defekte führen dürfen, da sonst die numerisch positionierten Fräs- und Auffüllwerkzeuge die Fehlstellen verfehlen bzw. nur ungenau flicken und damit zu Paneelen niedriger Qualität führen.

Wie in Fig. 2a gezeigt, werden heute solche Systeme vorwiegend so konstruiert, dass die Kamera 21 das Paneel 11 aus einer senkrechten Richtung unter einem Winkel von ungefähr β = 90 Grad optisch erfasst. Die Schwerpunkt-Koordinaten $[Xm, Ym]$ des Defektes ändern sich nach Fig. 2a nicht, wenn die Paneeloberfläche sich lokal am Ort des Defekts, wie in Fig. 2b gezeigt, etwa infolge einer Wölbung um den Betrag dZ erhöht, so daß solche Systeme eine gute geometrische Genauigkeit auch bei nicht ebenen Paneelen aufzeigen.

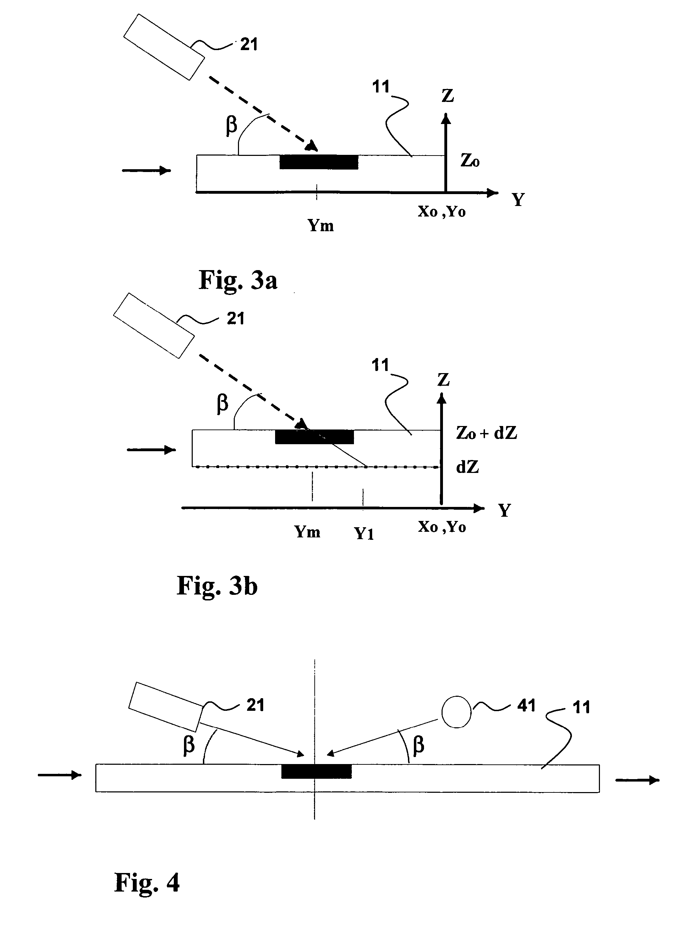

Dieser Vorteil wird allerdings, wie in Fig. 3a gezeigt, geschmälert durch die wesentlich geringere Empfindlichkeit der Detektion von Glanzstellen, rauhen Stellen und ähnlichen Oberflächenfehlern, welche wesentlich kontrastreicher aus einer Aufnahmerichtung mit einem flachen Aufnahmewinkel β abzubilden sind. Ändert sich allerdings die Höhe der Paneeloberfläche um einen Betrag dZ, beispielsweise durch eine Wölbung, so verschiebt sich im Bild der Kamera 21 der Schwerpunkt des Defektes auf Y1; es entsteht damit ein Positionsfehler

$$(1) \quad \Delta Y = (Y1-Ym) = dZ / \tan \beta$$

welcher sehr stark von der Höhendifferenz abhängt. Dem Flickroboter werden dadurch ohne Korrektur grob falsche Schwerpunktskoordinaten übertragen.

In Weiterbildung der Erfindung wird daher die sich aus den unkorrigierten Bilddaten ergebende Lage eines Defekts oder eines Konturpunkts um eine Verschiebung $\Delta Y = dZ / \tan(\beta)$ korrigiert, wobei $\Delta Y$ eine Verschiebung entlang der Oberfläche in Richtung auf den optischen Bildaufnehmer zu, dZ die ermittelte Höhenabweichung in Richtung senkrecht zur Oberfläche des Paneels und β den Winkel bezeichnen, unter welchem der optische Bildaufnehmer auf die Oberfläche eines nicht höhenverschobenen Paneels blickt.

Da sich die Koordinaten aller Konturpunkte des Defektes ebenfalls verschieben, ändert sich auch die Form des Defektes und damit eine wichtige Charakterisierung für den automatischen Flickprozess.

Bedenkt man weiterhin, dass eine symmetrische Hellfeld-Anordnung wie in Fig. 4 skizziert, mit sehr flachem Winkel β von im Bereich von 10 - 20 Grad besonders empfindlich arbeitet, so erkennt man, dass die gewünschte hohe Detektionsempfindlichkeit dieser Anordnung mit einer nicht akzeptalen geometrischen Ungenauigkeit in der Defektlage infolge unvermeidlicher Unebenheiten der Paneel erkauft wird.

Wie in Fig. 2a und 2b in einer Seitenansicht skizzenhaft verdeutlicht, wird bei einer weitgehend senkrechten Ausrichtung der Kamera (bezogen auf die Paneeloberfläche 11) die gewünschte Unabhängigkeit der Ym-Lage des Defektes von der Höhe (der Z-Koordinate) der inspizierten Fläche erreicht. Auch bei einer um die Höhe dZ angehobenen Paneeloberfläche wird die des Fehlers Ym im wesentlichen unverändert sein.

Allerdings wird diese Robustheit gegen Höhenänderungen und Welligkeiten der Paneeloberfläche durch eine geringe Empfindlichkeit bei der Erkennung von Oberflächenfehlern erkauft. Die Beleuchtung 22 und bildgebende Erfassung 21 aus einem relativ zur Paneelebene steilen Winkel von ca. 60 bis 90 Grad bildet nur kontrastreiche Fehler gut ab. Schwache, durch Oberflächeneffekte wie lokale Rauhigkeit, Rattermarken, Glanzstellen, kleine Beulen oder Eindrücke, Risse, offene Leimkanten zwischen beispielsweise Parkettlamellen usw. werden mit einer solchen Anordnung nicht erkannt.

Würde hingegen die Paneel-Oberfläche nach Fig. 3 a) und c) von einer schräg unter dem Winkel β beobachtenden Kamera erfasst, so verschiebt sich die aus dem Kamerabild gemessene Defektposition Ym einer Fehlstelle bei einer sich um den Betrag dZ ändernden Paneel-Höhe, beispielsweise durch eine lokale Wölbung bezogen auf den Koordinaten-Nullpunkt $[X_0, Y_0]$ des Paneels, wobei die Verschiebung der oben angegebenen Gleichung (1) entspricht. Dem FlickRoboter wird damit eine fehlerhafte Position des auszufräsenden und aufzufüllenden Defektes übergeben.

Der Vorteil einer hohen Empfindlichkeit einer flachen Bildaufnahme für die Erkennung von Defekten wird damit erkauft mit einer niedrigen Robustheit gegen Höhenschwankungen der Paneeloberfläche und damit mit einer niedrigen geometrischen Genauigkeit der Lokalisierung dieser Defekte.

Fig. 4 verdeutlicht an einem praktischen Beispiel die Bedeutung dieser geometrischen Betrachtung. Bei einem für eine symmetrische und empfindliche Hellfeld-Anordnung zwischen Beleuchtung 41 und Kamera 21 üblichen flachen Winkeln von β~10 Grad würde eine Wölbung des Paneels an der Defektstelle um lediglich 5 mm bereits ein untragbarer Lagefehler $\Delta Y$ von 28 mm entstehen.

Die flache Bilderfassung verschiebt bei einer gewölbten Oberfläche nicht nur die Koordinaten des Schwerpunktes eines Defektes, sondern die gesamte Kontur des Defektes. Die Form des Defektes verändert sich daher ebenfalls mit der Paneelhöhe und damit auch die für die Beurteilung des Defektes wichtige Charakterisierungen (Form der Randkontur, Fläche) und die daraus abgeleiteten Flickstrategien. Ein kreisrunder Defekt erscheint bei einer Wölbung infolge der flachen Bildaufnahme als ein in Vorschubrichtung zur Ellipse verzerrter Kreis und würde daher dem Flickroboter das Einsetzen eines ellipsenförmigen Dübels vorschlagen und damit eine physikalisch und ästhetisch ungünstige Reparatur durchführen.

Zur Verdeutlichung des Erfindungsgedanken seien weiterhin noch folgende Abbildungen angesprochen:

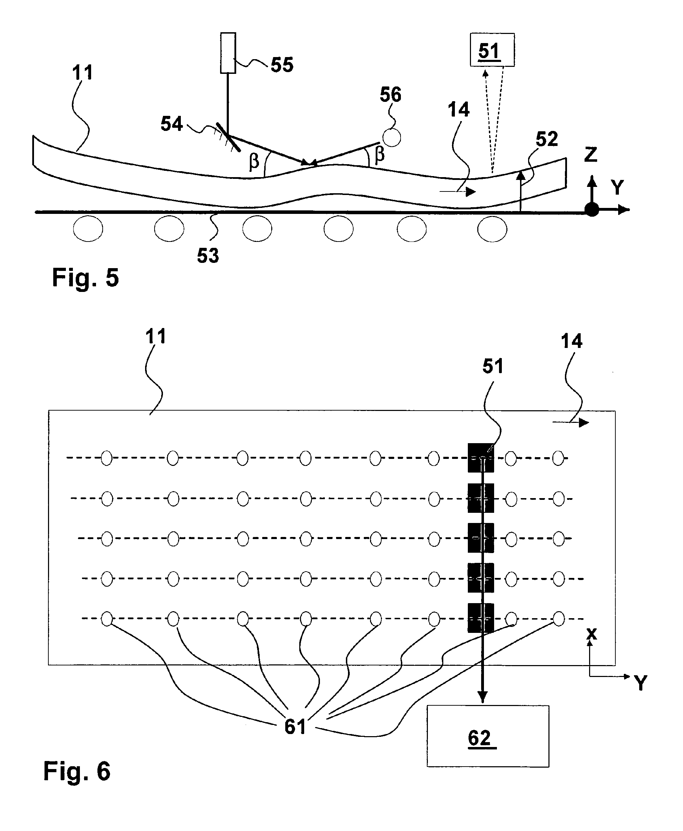

Fig. 5 zeigt schematisch in der Seitenansicht eine Anordnung eines punktförmig messenden Distanzsensors 51, welcher im Durchlauf in diskreten Abständen und in einer Längsspur die lokale Höhe 52 (bezogen auf die Grundebene) entlang der Vorschubrichtung 14 eines gewellten Paneels 11 bestimmt, einer Hellfeld- Anordnung mit einer unter einem flachem Winkel über einen Umlenkspiegel 54 die Paneel-Oberfläche zeilenförmig beobachtenden Kamera 55 und eine zeilenförmig beleuchtende Beleuchtung 56. Als Vorschubeinrichtung ist beispielhaft ein Förderband 53 vorgesehen, auf welchem das Paneel 11 aufgelegt ist.

Als Meßwertgeber für den oder die Distanzsensoren 51 können unter anderem punktförmig abtastende Laser-Triangulations-Entfernungs-Sensoren eingesetzt werden. Mit Hilfe mehrerer solcher Sensoren lässt sich ein ausreichend dichtes Gitter von Höhenmeßpunkten der Paneeloberfläche im Durchlauf erstellen. Auch sogenannte Distance-from-focus-Sensoren, oder Flugzeit-Sensoren können verwendet werden. Die Abtastung kann gemäß noch einer Ausführungsform auch mechanisch, also berührend erfolgen.

Die Paneeloberfläche wird mit einer empfindlichen Hellfeld-Anordnung im Durchlauf abgetastet, umfassend eine vorzugsweise zeilenförmig abtastende Kamera 55, welche beispielsweise zwecks kompakter Bauweise über den Umlenkspiegel 54 unter einem flachen Winkel $\beta$ die Paneeloberfläche erfasst, welche symmetrisch unter einem ebenfalls flachen Winkel durch die zeilenförmige Beleuchtungsquelle 56 beleuchtet wird. Vorzugsweise sind die Winkel von einfallendem Lichtstrahl und Blickrichtung der Kamera gleich. Durch die im voraus bekannte mechanische Anordnung von Distanzsensor zu Kamera-Beobachtungslinie ist die durch den Distanzsensor gemessene lokale Paneelhöhe Zm nunmehr bei jeder Bildaufnahme bekannt.

Erfindungsgemäß werden die an die Flickstation zu übertragende Lagekoordinaten des Defektes nach der Beziehung (1) korrigiert:

$$(2)\quad Ym(korr) \;=\; Ym \;+\; \Delta Y,$$

und

$$(3)\quad Xm(korr) \;=\; Xm$$

und damit die Lagefehler infolge einer nicht konstanten Höhe $Z_1$ der Paneeloberfläche beseitigt. Ym bezeichnet dabei die Y-Koordinate des Defekts im Allgemeinen, insbesondere des Defektschwerpunktes oder eines Defektkonturpunkts, wie sie aus dem Bild der aufnehmenden Kamera bei einem eben aufliegenden Paneel mit der Stärke $Z_0$ berechnet wird. Der Winkel $\beta$ ist der durch die feste mechanische Anordnung von Kamera zur Ebene der Paneeltransportvorrichtung bekannte Betrachtungswinkel.

Das erfindungsgemäße Verfahren ist damit in der Lage, sowohl die Auswirkungen der lokalen Höhenänderung $Z_1$ der Paneeloberfläche infolge von Welligkeit und Wölbung als auch die Auswirkung einer veränderlicher Paneelstärke auf die Genauigkeit der Positionskoordinaten von Defekten im Paneel-bezogenen Koordinatensystem $[X_0, Y_0]$ zu korrigieren.

Fig. 6 zeigt in der Aufsicht eine beispielhafte Anordnung von insgesamt fünf Distanzsensoren, welche an diskreten Punkten die lokale Paneelhöhe Zm erfassen. Die Erfassung der lokalen Höhe der Paneeloberfläche erfolgt im Durchlauf, im dargestellten Beispiel dabei an 30 diskreten Stellen 61. Die Distanzsensoren 51 sind zweckmäßig quer zur Vorschubrichtung 14 aufgereiht angeordnet.

Dieses so erhaltene diskrete Gitter von Höhenstützwerten kann dann durch eine zweidimensionale Interpolation (beispielsweise durch Bi-Splines) in ein stetige zweidimensionale Höhenkarte der Paneeloberfläche 63 mit Hilfe der Korrektureinheit 62 umgerechnet werden, so daß für jede beliebige Defektposition [Xm,Ym] die Lagekorrektur berechnet werden kann.

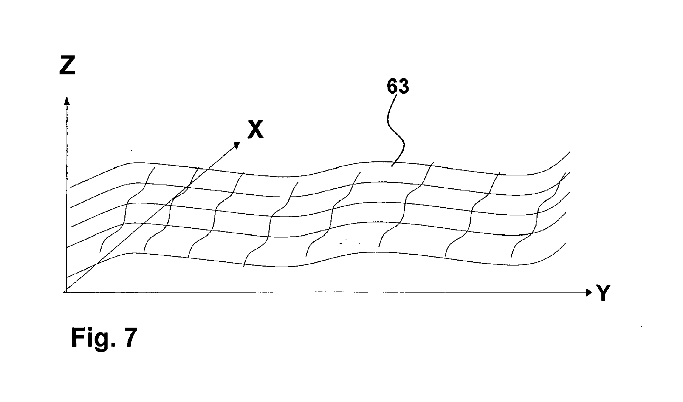

Das anhand der an den Stellen 61 gemessenen Höhen von der Korrektureinheit 62 durch eine zweidimensionale Interpolation berechnete, stetige niederfrequente Höhenprofil 63 der Oberfläche ist in Fig. 7 dargestellt.

Erfindungsgemäß wird diese Korrektur nicht nur auf den Schwerpunkt des Defektes angewandt, sondern ebenso auf die Konturpunkte des Defektes, so dass auch die höhenabhängige Verformung des Defektes im aufgenommenen Bild infolge einer Paneelwölbung korrigiert werden kann.

Die Bestimmung der Paneelhöhe durch punktweise messende Distanzsensoren sind nur beispielhaft zu verstehen.

Der Erfindungsgedanken ist auch nicht auf Holzpaneele beschränkt, sondern umfasst automatische Flickstationen für alle denkbaren Materialien wie Natursteine, Marmor, mehrlagige Paneele usw. bei welchen häufig lokale Fehler vorhanden sind und sich aufgrund des hohen Wertes eine automatisches Flicken lohnt.

Die Beobachtung der lokalen Lichtreflexion in einer Hellfeld-Anordnung ist ebenfalls beispielhaft als eine optische Abtastung zu verstehen, bei welcher Prinzipbedingt eine veränderliche Paneelhöhe zu einem Lagefehler bei den ermittelten Defekten führt. Auch andere, unter einem schrägen Winkel die Oberfläche erfassende optische Verfahren wie beispielsweise eine Lichthof-Kamera fallen unter den allgemeinen Erfindungsgedanken, solange diese Bildaufnahmeanordnung bei Höhenänderungen der Oberfläche zu Lagefehler der erkannten Defekte führen.

Der Erfindungsgedanke umfasst auch sog. "multisensorielle" optische Abtaster für Paneeloberflächen, bei welchen mehrere Kamera-/Beleuchtungssysteme zur besseren Defekterkennung kombiniert werden, sofern bei mindestens einem dieser Systeme eine sich verändernden Paneelhöhe zu einer Fehlmessung der Defektposition führt.

Der Begriff "Paneel" ist im Rahmen dieser Anmeldung nicht materialspezifisch zu verstehen, insbesondere nicht auf Naturholz beschränkt. Der Begriff soll alle vorwiegend flachen, beziehungsweise plattenförmigen Materialflächen bezeichnen, bei welchen lokalen physikalische oder ästhetische Fehler auftreten können und diese durch eine automatische Flickstation repariert werden sollen.

Wenn auch erfindungsgemäß die technisch wichtigste Abtastung der Paneeloberfläche im Durchlauf durch zeilenweise arbeitende Bildsensoren erfolgt, so ist der Erfindungsgedanke hierauf nicht beschränkt. Auch die bildhafte Abtastung durch numerisch über dem ruhenden Paneel positionierte Bildgeber/Beleuchtungs/Höhenmeß-Anordnungen sind Teil des Erfindungsgedanken.

Zusammengefasst besteht der Erfindungsgedanke darin, hochempfindliche Bildaufnahme-/Beleuchtungsanordnungen zur Erkennung von Defekten auf automatisch zu flickenden, im wesentlichen flachen Produkten auch dann einzusetzen, wenn durch das Aufnahme-/Beleuchtungsprinzip bedingt Höhenänderungen der Oberfläche infolge von Wölbung, Welligkeit, lokal veränderlicher Materialstärke usw. zu Fehler in den erkannten Defekt-Positionen führen und diese Positions-Fehler dadurch zu korrigieren, indem die lokalen Höhenänderungen gegenüber einer Soll-Höhe durch ein Höhenmeßsystem zusätzlich erfasst werden und mit diesen lokalen Höhenwerten und den bekannten geometrischen Anordnungen von Bildaufnahme/Beleuchtungssystem Korrekturwerte zur geometrischen Korrektur der aus dem Kamerabild gewonnenen Defektpositionen berechnet werden und diese korrigierten Defektpositionen zur Steuerung der automatischen Flickstation eingesetzt werden.

**Patentansprüche**

1.  Verfahren zum automatischen Flicken von Defekten in Paneelen, bei welchem die Defekte mit Hilfe eines optischen Bildaufnehmers erkannt und diese Stellen mit Hilfe von zumindest einem rechnergesteuerten Werkzeug ausgefüllt oder beseitigt werden, wobei der optische Bildaufnehmer die Oberfläche des Paneels unter schrägem Winkel betrachtet, wobei das rechnergesteuerte Werkzeug anhand der von dem optischen Bildaufnehmer ermittelten Defekt-Positionen angesteuert wird, wobei aus dem oder den erzeugten Bildern mittels einer rechnergestützten automatisierten Bildverarbeitung die zu flickenden Defekte erkannt und lokalisiert werden, während das Paneel lateral entlang einer Vorschubrichtung relativ zum optischen Bildaufnehmer vorbeigeführt wird,
    wobei zusätzlich eine Höhenmessung der Lage der Oberfläche des Paneels mittels einer Höhenmesseinrichtung durchgeführt und die lokale Höhe der Defekte anhand der Höhenmessung bestimmt wird, und wobei die anhand der automatisierten Bildverarbeitung ermittelten lateralen Positionen der Defekte mittels der bestimmten lokale Höhe der Defekte korrigiert, und das rechnergesteuerte Flickwerkzeug mit diesen korrigierten Positionsinformationen der Defekte angesteuert wird.

2.  Verfahren gemäß dem vorstehenden Anspruch, wobei die zu flickenden Defekte erkannt und lokalisiert werden, während das Paneel lateral entlang einer Vorschubrichtung relativ zum optischen Bildaufnehmer vorbeigeführt wird.

3.  Verfahren gemäß Anspruch 1 oder 2, wobei die zu flickende Oberfläche von mindestens einer Hellfeld-Anordnung von Bildaufnehmer und Beleuchtungsquelle optisch unter schrägem Winkel bezogen auf Oberfläche des Paneels erfasst wird.

4.  Verfahren nach einem der vorstehenden Ansprüche, **dadurch gekennzeichnet dass** die lokale Höhe der zu flickenden Oberfläche im Durchlauf mit einer Anzahl von optischen Distanzsensoren an diskreten Positionen der zu flickenden Oberfläche erfasst wird.

5.  Verfahren nach Anspruch 1 oder 2, **dadurch gekennzeichnet dass** über eine zweidimensionale Interpolation aus diskreten Höhenmeßwerten eine stetige Höhenkarte der zu flickenden Oberfläche ermittelt wird und die interpolierten Höhenwerte dieser Höhenkarte zur Lagekorrektur der Defekte verwendet werden.

6.  Verfahren nach einem der vorstehenden Ansprüche, **dadurch gekennzeichnet dass** die lokale Höhe der zu inspizierenden Oberfläche mit Hilfe mindestens eines die zu flickende Oberfläche im Durchlauf linienförmig quer zur Vorschubrichtung des Paneels in der Höhe ausmessenden Distanzsensors bestimmt wird.

7. Verfahren nach einem der vorstehenden Ansprüche, **dadurch gekennzeichnet dass** die zu flickende Oberfläche durch einen oder mehrere zeilenweise abtastende Bildsensoren im Durchlauf erfasst wird.

8. Verfahren nach einem der vorstehenden Ansprüche, **dadurch gekennzeichnet dass** die zu flickende Oberfläche durch einen zeilenweise oder linienförmig die Oberfläche beleuchtende Lichtquelle beleuchtet wird.

9. Verfahren nach einem der vorstehenden Ansprüche, **dadurch gekennzeichnet dass** die zu flickende Oberfläche durch mehrere kombinierte Bilderfassungs-und Beleuchtungsanordnungen zur Erkennung von zu flickenden Defekte abgetastet wird und die aus den Bilddaten ermittelten Positionsdaten der Defekte und/oder der Defektkonturpunkte lediglich oder insbesondere bei einer solchen Anordnung mit Hilfe von zusätzlich gemessenen Höhenwerten korrigiert werden, bei welcher die Bilderfassungs-/Beleuchtungsanordnung prinzipbedingt zu Positionsfehlern der Defekte und/oder Defektkonturpunkte bei einer sich ändernden Höhe der Paneeloberfläche führt.

10. Verfahren gemäß einem der vorstehenden Ansprüche,
**dadurch gekennzeichnet, dass** die rechnergestützte automatisierte Bildverarbeitung eine Charakterisierung der ermittelten Defekte, insbesondere hinsichtlich zumindest einer der Eigenschaften Form, Fläche, Farbe vornimmt.

11. Verfahren gemäß einem der vorstehenden Ansprüche,
**dadurch gekennzeichnet, dass** die rechnergestützte automatisierte Bildverarbeitung die Kontur eines Defekts bestimmt und die Lage der Konturpunkte des Defekts anhand der mittels der bestimmten lokale Höhe des Defekts korrigiert wird.

12. Verfahren gemäß einem der vorstehenden Ansprüche,
**dadurch gekennzeichnet, dass** die sich aus den unkorrigierten Bilddaten ergebende Lage eines Defekts oder eines Konturpunkts um eine Verschiebung $\Delta Y = dZ / \tan(\beta)$ korrigiert wird, wobei $\Delta Y$ eine Verschiebung entlang der Oberfläche in Richtung auf den optischen Bildaufnehmer zu, dZ die ermittelte Höhenabweichung in Richtung senkrecht zur Oberfläche des Paneels und b den Winkel bezeichnen, unter welchem der optische Bildaufnehmer auf die Oberfläche eines nicht höhenverschobenen Paneels blickt.

13. Vorrichtung zum automatischen Flicken von Defekten in Paneelen, insbesondere ausgebildet zur Durchführung eines Verfahrens nach einem der vorstehenden Ansprüche, umfassend

- zumindest einen optischen Bildaufnehmer, der so angeordnet ist, dass dessen Blickrichtung schräg unter einem Winkel zur Oberfläche eines zu untersuchenden Paneels liegt,
- zumindest einen Höhensensor zur Ermittlung der Höhenlage der Oberfläche des Paneels,
- eine Recheneinrichtung zur automatischen Erkennung von Defekten anhand der vom zumindest einen optischen Bildaufnehmer gelieferten Bilddaten und zur Ermittlung der lateralen Position der Defekte, wobei die Recheneinrichtung dazu eingerichtet ist, anhand der vom Höhensensor gelieferten Meßwerte die Höhenlage der Defekte zu bestimmen und die laterale Position der Defekte anhand der bestimmten Höhenlage zu korrigieren,
- ein Flickwerkzeug, welches von der Recheneinrichtung angesteuert ist, um die Defekte an deren durch die Höhenlage korrigierten lateralen Positionen auszubessern.

14. Vorrichtung gemäß dem vorstehenden Anspruch, umfassend eine Beleuchtungseinrichtung, welche so gegenüber dem optischen Bildaufnehmer angeordnet ist, dass die zu flickende Oberfläche vom optischen Bildaufnehmer im Hellfeld des von der Oberfläche reflektierten Strahls betrachtet wird.

15. Vorrichtung gemäß einem der beiden vorstehenden Ansprüche, **dadurch gekennzeichnet, dass** der optische Bildaufnehmer so angeordnet ist, dass dieser die Oberfläche eines Paneels unter einem Winkel von 5 bis 60, vorzugsweise von 8 bis 30 Grad bezogen auf die Oberfläche betrachtet.

16. Vorrichtung gemäß einem der vorstehenden Ansprüche,
**dadurch gekennzeichnet, dass** das Flickwerkzeug eine Einrichtung zum Aushöhlen, vorzugsweise Ausfräsen, Ausstanzen, oder Ausbohren von Defekten und eine Einrichtung zum Ausfüllen des ausgehöhlten Bereichs durch ein Flickmittel, vorzugsweise durch Spachtelmasse oder Dübel umfasst.

EP 2 239 116 A2

**Fig. 1**

**Fig. 2a**

**Fig. 2b**

**Fig. 3a**

**Fig. 3b**

**Fig. 4**

**Fig. 5**

**Fig. 6**

EP 2 239 116 A2

Z

X

63

Y

Fig. 7

13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4614555 A
- US 4984172 A